# EUROPEAN PATENT APPLICATION

(11) **EP 4 216 229 A1**
(43) Date of publication of application: **26.07.2023**
(21) Application number: 22153237.7
(22) Date of filing: 25.01.2022
(51) Int. Cl.: G16H 20/40, G16H 30/40, G16H 40/20

(54) **SUBSCRIPTION AND RETRIEVAL OF MEDICAL IMAGING DATA**

(71) Applicant: Siemens Healthcare GmbH, 91052 Erlangen (DE)
(72) Inventor: Boettger, Thomas, 91054 Erlangen (DE); Spitzner, Christian, 91301 Forchheim (DE)

(57) **Abstract**

Techniques of facilitating operation of a user node to obtain medical imaging data of a patient are disclosed. At least one instance of medical imaging data of the patient is obtained based on semantic information associated with the at least one instance of the medical imaging data. The semantic information is obtained, from a first data repository of a network, based on patient information of the patient.

## Description

Various examples of the disclosure relate to facilitating operation of a user node to automatically obtain semantic information associated with medical imaging data of a patient. Various examples of the disclosure relate to obtaining medical imaging data of a patient.

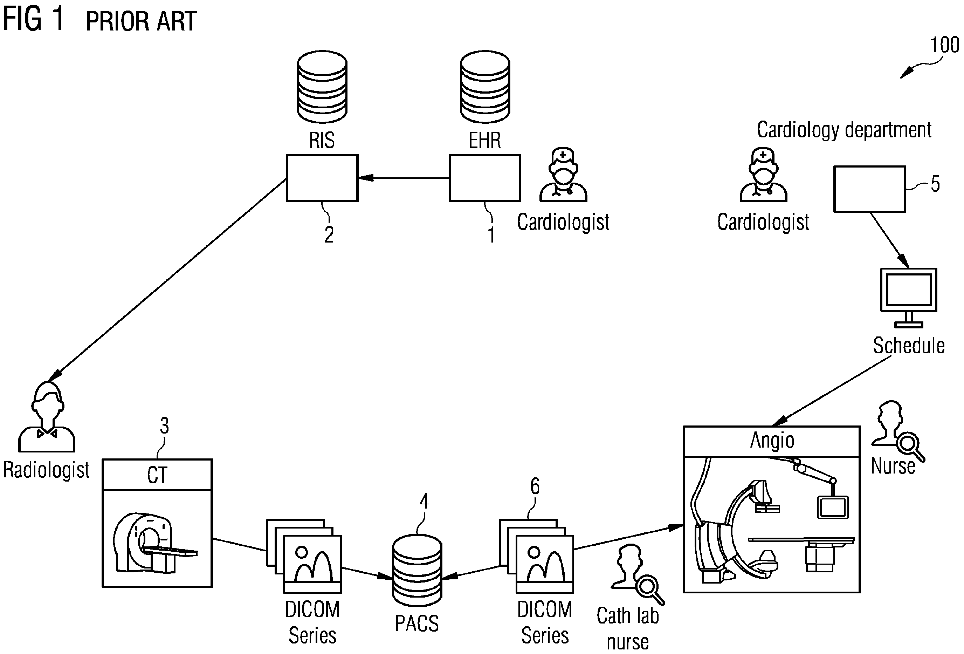

A variety of imaging techniques such as X-ray radiography, ultrasound, computed tomography (CT), nuclear medicine including positron emission tomography (PET), fluoroscopy, and magnetic resonance imaging (MRI) are being used to diagnose or treat diseases in current clinical practices. In general, a workflow of a radiology examination may include the following activities: placing an order, scheduling the order, acquiring imaging data through modalities, e.g., X-ray scanner, CT scanner, PET scanner, or MRI scanner, storing the acquired imaging data, viewing the acquired imaging data and producing a radiology report. An example of a radiology examination is illustrated in connection with FIG. 1.

FIG. 1 schematically depicts a workflow of a Coronary Computed Tomography Angiography (CTA) examination according to the prior art.

At step 1, a cardiologist orders the coronary CAT examination, for example via an Electronic health record (EHR) system or a Health Information System (HIS), by sending a request to the Radiological Information System (RIS).

At step 2, upon receiving the request sent from the EHR system, the RIS schedules the coronary CTA examination.

Then, at step 3, a radiologist carries out the coronary CTA examination based on RIS's schedules, and accordingly, CTA imaging data are acquired.

At step 4, the acquired CTA imaging data are transmitted to and stored in a data repository, e.g., a Picture Archiving and Communication System (PACS), according to a standard, such as Digital Imaging and Communications in Medicine (DICOM). After the coronary CTA examination, the radiologist reviews/studies the acquired CTA imaging data and produces a radiology report, e.g., a free-text radiology report or a structured radiology report. For example, the radiologist may obtain, via a user node connected to the PACS, the CTA imaging data from the PACS, and compile the radiology report. After adding a signature to the radiology report, the radiologist provides the signed radiology report to the cardiologist via the EHR system. Usually, the (signed) radiology report is an electronically readable Portable Document Format (PDF) document which may be linked with the original order of the coronary CTA examination and is archived in the EHR system.

If the cardiologist wants to access information beyond the signed radiology report (i.e., some semantic information associated with the medical imaging data or further medical imaging data), the cardiologist has to call the radiology department. In some hospitals/institutions, the cardiologist does have access to the PACS directly. However, the PACS has been designed for archiving imaging results and the DICOM standard optimizes this use case. The PACS has not been designed for clinical information exchange in a clinical workflow which goes beyond radiology. Cardiology staff usually struggles when trying to identify the "right images or imaging data" in the PACS directly.

For example, referring to FIG. 1 again, the cardiologist may place an order for a percutaneous coronary intervention of a patient at step 5. Before carrying out the percutaneous coronary intervention as scheduled, a catheter lab nurse has to browse the right CTA images or CTA imaging data for assisting the percutaneous coronary intervention directly from the patient record view, which is purely image-focused and the catheter lab nurse has to browse a list of DICOM studies & series, select one specific series and look at it and interpret what he/she sees. The whole procedure is quite time-consuming and the catheter lab nurse may not obtain the right images or imaging data in the end. Further, there may be a 3D coronary key summary, i.e., a 3D visualization of the coronary, obtained by post-processing the acquired coronary CTA available in the PACS, however, it is also time-consuming and may be impossible for the catheter lab nurse to obtain such a 3D coronary key summary relying on DICOM only.

FIG. 1 is only an illustration of one possible example; the same or similar technical problems confront other clinical staff, e.g., oncologists, surgeons, neurologists, and so on. Consequently, great imaging results acquired in radiology cannot be efficiently integrated into various clinical workflows along various patient pathways.

Accordingly, there is a need for advanced techniques that mitigate or overcome the above-identified drawbacks or restrictions. There is a need for advanced techniques of automatically obtaining additional information or data associated with a patient.

This need is met by the features of the independent claims. The features of the dependent claims define embodiments.

Hereinafter, techniques of obtaining, via a user node, information associated with a patient will be described. In some examples, medical imaging data can be obtained. Alternatively or additionally, semantic information associated with the patient can be obtained.

A computer-implemented method of operating a user node is provided. The computer-implemented method includes obtaining patient information of a patient and obtaining, from a first data repository of a network, semantic information associated with at least one instance of medical imaging data of the patient based on the patient information. The computer-implemented method further includes obtaining the at least one instance of the medical imaging data based on the semantic information.

A computer program or a computer-program product or a computer-readable storage medium including program code is provided. The program code can be loaded and executed by at least one processor. Upon loading and executing the program code, the at least one processor performs a computer-implemented method of operating a user node. The computer-implemented method includes obtaining patient information of a patient and obtaining, from a first data repository of a network, semantic information associated with at least one instance of medical imaging data of the patient based on the patient information. The computer-implemented method further includes obtaining the at least one instance of the medical imaging data based on the semantic information.

A device includes at least one processor and at least one memory. The at least one processor is configured to load program code from the at least one memory and execute the program code. Upon executing the program code, the at least one processor is configured to obtain patient information of a patient and obtain, from a first data repository of a network, semantic information associated with at least one instance of medical imaging data of the patient based on the patient information. The at least one processor is further configured to obtain the at least one instance of the medical imaging data based on the semantic information.

It is to be understood that the features mentioned above and those yet to be explained below may be used not only in the respective combinations indicated, but also in other combinations or in isolation without departing from the scope of the invention.
FIG. 1 schematically illustrates an exemplary workflow of a coronary CTA examination according to the prior art.
FIG. 2 schematically illustrates details with respect to a system 1000 according to various examples.
FIG. 3 is a flowchart of a method according to various examples.
FIG. 4 is a block diagram of a device according to various examples.

Some examples of the present disclosure generally provide for a plurality of circuits or other electrical devices. All references to the circuits and other electrical devices and the functionality provided by each are not intended to be limited to encompassing only what is illustrated and described herein. While particular labels may be assigned to the various circuits or other electrical devices disclosed, such labels are not intended to limit the scope of operation for the circuits and the other electrical devices. Such circuits and other electrical devices may be combined with each other and/or separated in any manner based on the particular type of electrical implementation that is desired. It is recognized that any circuit or other electrical device disclosed herein may include any number of microcontrollers, a graphics processor unit (GPU), integrated circuits, memory devices (e.g., FLASH, random access memory (RAM), read only memory (ROM), electrically programmable read only memory (EPROM), electrically erasable programmable read only memory (EEPROM), or other suitable variants thereof), and software which co-act with one another to perform operation(s) disclosed herein. In addition, any one or more of the electrical devices may be configured to execute a program code that is embodied in a non-transitory computer readable medium programmed to perform any number of the functions as disclosed.

In the following, embodiments of the invention will be described in detail with reference to the accompanying drawings. It is to be understood that the following description of embodiments is not to be taken in a limiting sense. The scope of the invention is not intended to be limited by the embodiments described hereinafter or by the drawings, which are taken to be illustrative only.

The drawings are to be regarded as being schematic representations and elements illustrated in the drawings are not necessarily shown to scale. Rather, the various elements are represented such that their function and general purpose become apparent to a person skilled in the art. Any connection or coupling between functional blocks, devices, components, or other physical or functional units shown in the drawings or described herein may also be implemented by an indirect connection or coupling. A coupling between components may also be established over a wireless connection. Functional blocks may be implemented in hardware, firmware, software, or a combination thereof.

Various techniques disclosed herein generally relate to facilitating operation of a user node - e.g., a PC or workstation in a hospital - to obtain medical imaging data of a patient. At least one instance of medical imaging data of the patient is obtained based on semantic information associated with the at least one instance of the medical imaging data.

The semantic information may be obtained, from a first data repository of a network, based on patient information of the patient. Alternatively or additionally, it would be possible that the semantic information is, at least partially, determines ad hoc.

According to the disclosure, the medical imaging data may depict an anatomical target region of a patient, e.g., the heart, the liver, the brain, or a part thereof. Various kinds and types of medical imaging data may be automatically obtained based on semantic information associated with the medical imaging data. As a general rule, it would be possible that 2-D images in the spatial domain or raw data in K-space are obtained. It would be also possible 3-D depth data, e.g., point clouds or depth maps, are obtained. Voxel data structures may be obtained, e.g., from CT scanner or MRI scanner. Time-varying data, where one dimension stores an image or volume representation at different points in time, may be obtained as well.

As a general rule, various kinds and types of medical imaging data can be subject to the techniques described herein. To give a few examples, it would be possible to use MRI imaging data, e.g., raw data in K-space or pre-reconstructed images. Another example would pertain to CT imaging data, e.g., projection views or pre-reconstructed images. Yet another example would pertain to PET imaging data. Other examples include ultrasound images or a combination of at least two of the medical imaging data outlined above.

In general, medical imaging data may include raw data generated by a medical imaging scanner, e.g., CT, MRI, or PET, reconstructed 2-D or 3-D images, a video of reconstructed images, images or videos obtained after post-processing. For example, the images or videos obtained after post-processing may include at least one of visualization of the medical imaging data, segmentations of the medical imaging data, denoised images of the medical imaging data, and so on. The type of the images or videos obtained after post-processing relies on post-processing techniques. The visualization of the medical imaging data could be encoded in predefined image types, e.g., VRT (GDAL Virtual Format), MPR (Multiplanar reformation or reconstruction), MIP (Maximum intensity projection), Curved Planar, and Straight Planar.

According to examples, by automatically obtaining medical imaging data, various use cases can be facilitated. According to examples, it would be possible to post-process the medical imaging data. For instance, certain characteristics of the anatomy depicted by the medical imaging data could be extracted using the post-processing. In this disclosure, various post-processing techniques may be used to perform post-processing of the medical imaging data, such as image enhancement, image segmentation, object detection, and so on. The post-processing may be based on conventional rule-based methods, e.g., independent component analysis or principal component analysis, or based on artificial intelligence (AI) methods, e.g., machine-learning (ML) algorithms.

As a general rule, various kinds and types of ML algorithms can facilitate the techniques described herein. For instance, it would be possible to use a deep neural network, e.g., a convolutional neural network having one or more convolutional layers performing convolutions between the input data and a kernel. It would also be possible to use a support vector machine, to give just a few examples. A U-net architecture may be used, see, e.g., Ronneberger, O., Fischer, P. and Brox, T., 2015, October. U-net: Convolutional networks for biomedical image segmentation. In International Conference on Medical image computing and computer-assisted intervention (pp. 234-241). Springer, Cham. Further, a landmark detection ML algorithm may be used, e.g., Ghesu et al., Multi-Scale Deep Reinforcement Learning for Real-Time 3D-Landmark Detection in CT Scans, IEEE PAMI 2018.

In the examples described herein, the ML algorithm can be configured to perform various tasks when (post-)processing the medical imaging data. An ML algorithm can also implement combination of such tasks as presented hereinafter. For instance, the ML algorithm could be configured to perform diagnosis of diseases, such as neurological/psychiatric diseases, cancer, hepatitis diseases, and etc. Further, the ML algorithm could be configured to perform a segmentation of medical imaging data. For instance, it would be possible to segment predefined anatomical features. In a further example, the ML algorithm could be configured to perform an object detection. For instance, a bounding box could be drawn around a predefined object that is detected in the medical image data. Predefined objects could be predefined anatomical features, e.g., certain organs or vessels, a tumor site, etc.. It would also be possible to detect anomalies. Yet a further task would be filtering or denoising. For instance, it would be possible to remove artifacts from the imaging modality, e.g., blurs or flare spots. Background noise could be suppressed or removed. Yet a further task would be up-sampling, to thereby increase a resolution of the medical imaging data. Yet a further task of the ML algorithm could be image reconstruction. For instance, for magnetic resonance imaging data, the raw imaging data is available in K-space. Sometimes, the K-space is undersampled (e.g., with respect to a certain field-of-view, considering Nyquist's theorem), i.e., a direct Fourier transform would result in aliasing artifacts. In such scenarios, image reconstruction can be implemented using an appropriate ML algorithm. For example, techniques are known where an unrolled neural network is used; here, a regularization operation can be implemented using the ML algorithm. As will be appreciated from the above, the particular type of the ML algorithm is not germane for the functioning of the techniques described herein. Rather, various kinds and types of ML algorithms can facilitate the techniques described herein, i.e., images or videos obtained after post-processing, e.g., using various ML algorithms, can be automatically integrated into various clinical workflows along various patient pathways and thereby facilitate precise diagnosis and/or treatment of the patient.

According to this disclosure, patient information - facilitating retrieval of further information or data associate with the patient - may include demographic and administrative information, such as at least one of a name (first and last name), date of birth, a gender, a race, the current address, a phone number, an email address, an identity number, an issuer of the identity number, an original unique identity number, an insurance number. Optionally or additionally, patient information may include names of existing diseases of the patient or other historical diagnoses. Such patient information can be informed by the patient or obtained, via a smart card reader, by communicating with a smart card storing the patient information, such as an insurance card.

According to the disclosure, semantic information associated with at least one instance of medical imaging data of a patient may describe meaningful image objects and their mutual relations in the at least one instance of medical imaging data. The semantic information may further include at least one of anatomical information of the patient, visualization information of the patient, medical measurements of the patient, imaging protocol information for acquiring the medical imaging data, information of imaging equipment for acquiring the medical imaging data, and diagnosis information. Additionally or optionally, the semantic information may include an identifier indicating a storage location of the at least one instance of the medical imaging data on the network, e.g., a Uniform Resource Locator (URL). For example, each instance of the medical imaging data may have a URL and the semantic information may include multiple URLs linked to different parts of the semantic information. As an example, the semantic information recites that "there are two plaques in the left coronary artery" and there may be two URLs respectively linked to medical imaging data depicting the two plaques. According to various examples, the semantic information could be structured based on a pre-configured data model, e.g., FHIR. For example, according to a specific data model, the semantic information including corresponding URLs of medical imaging data may be structured using a markup language, e.g., Extensible Markup Language (XML) which is both human-readable and machine-readable. Therefore, it is possible to obtain the identifier indicating the storage location of the at least one instance of the medical imaging data by parsing the semantic information based on the pre-configured data model.

According to various examples, image analysis can be utilized to extract semantic information associated with medical imaging data. Image analysis can be broadly classified in two categories: human visual cortex-based image analysis and computer image analysis. The conventional activities of reviewing/studying, and interpreting medical imaging data involving radiologists correspond to human visual cortex based image analysis. On the other hand, computer image analysis may cover the fields of computer or machine vision, and medical imaging, and make use of pattern recognition, digital geometry, and signal processing. Computer image analysis may be further classified as digital image analysis and object based image analysis. It is a quantitative or qualitative characterization of two-dimensional or three-dimensional digital images.

In general, various computer image analysis techniques can be utilized to automatically and accurately extract semantic information associated with specific medical imaging data of a patient. For example, techniques with respect to multiscale mode for image analysis of brain tumor can be used, see, Bauer, Stefan, et al. "Multiscale modeling for image analysis of brain tumor studies." IEEE transactions on biomedical engineering 59.1 (2011): 25-29. As another example, medical image analysis techniques may be employed for analysis and visualization of medical image data in an appropriate way, see, Ritter, Felix, et al. "Medical image analysis." IEEE pulse 2.6 (2011): 60-70. Accordingly, an overview of a radiology examination may be obtained from the visualization of the medical imaging data acquired during the radiology examination. Further, AI-based image analysis methods can be used, such as ML algorithms for extracting semantic information associated with medical imaging data. Some examples of such AI-based medical imaging data analysis methods are as follows: Shen, Dinggang, Guorong Wu, and Heung-Il Suk. "Deep learning in medical image analysis." Annual review of biomedical engineering 19 (2017): 221-248; Taghanaki, Saeid Asgari, et al. "Deep semantic segmentation of natural and medical images: a review." Artificial Intelligence Review 54.1 (2021): 137-178; Kar, Mithun Kumar, Malaya Kumar Nath, and Debanga Raj Neog. "A Review on Progress in Semantic Image Segmentation and Its Application to Medical Images." SN Computer Science 2.5 (2021): 1-30.

In general, computer image analysis techniques may start with segmentation, where an individual image is decomposed into segments. After segmentation, clustering may be carried out, in which determinate clusters and gasps within each of segments and assign semantic terms to each cluster or gasp. After assigning appropriate fragment or fragments to each semantic term, analysis of actual fragments may be carried out to get appropriate quantitative value for each semantic term. Finally, appropriate semantic networks and partial semantic functions and values may be developed. A final semantic function and its semantic value may assign to the analyzed image. Clusters could create segments and the image as a whole. Normally, combinations of number of image processing techniques may be used for semantic analysis of image content, and could create and update of image databases. The computer image analysis techniques mine semantic pixel information, semantic feature information and semantic fact and figure about image.

According to various examples, preliminary semantic information associated with medical imaging data may be first extracted using computer image analysis techniques and then may be revised by radiologists to obtain accurate semantic information associated with the medical imaging data.

According to various examples, the medical imaging data may include multiple instances (i.e., pieces) of the medical imaging data and each of or a small group of the multiple instances of the medical imaging data may include individual semantic information associated with each of or the small group of the multiple instances. For example, the medical imaging data may be reconstructed CT images and each slice of the reconstructed CT images may include a piece of semantic information extracted from respective slice of the reconstructed CT image. As another example, since neighboring images may share similar or the same semantic information, several, e.g., any of 2-20, images may share the same piece of semantic information extracted from corresponding neighboring images.

According to this disclosure, after extracting semantic information associated with medical imaging data, the extracted semantic information may be standardized based on pre-defined regulations to facilitate automatic processes using computers and interoperability among elements of healthcare system. The regulations may be standardized. For example, SNOMED Clinical Terms (CT) and/or RadLex radiology lexicon can be used to facilitate clinical documentation and reporting. Anatomical information of a patient may be encoded following the Radlex or SNOMED CT.

According to various examples, the (standardized) semantic information associated with medical imaging data may be structured based on a pre-configured data model, which explicitly determines the structure of semantic information. For example, the OMOP Common Data Model, the PICO model, or the FHIR (Fast Healthcare Interoperability Resources) data model. By structuring the semantic information based on a pre-defined data model, it is possible to facilitate interoperability between legacy health care systems, to make it easy to provide health care information to health care providers and individuals on a wide variety of devices from computers to tablets to cell phones and to allow third-party application developers to provide medical applications which can be easily integrated into existing systems.

For example, FHIR provides an alternative to document-centric approaches by directly exposing discrete data elements as services and thereby basic elements of healthcare like patients, admissions, diagnostic reports, and medications can each be retrieved and manipulated via their own resource URLs. FHIR is organized by resources (e.g., patient, observation). Such resources can be specified further by defining FHIR profiles (for example, binding to specific terminology, e.g., SNOMED CT or RadLex). A collection of profiles can be published as an implementation guide (IG), such as the U.S. Core Data for Interoperability (USCDI). FHIR is implemented on top of the HTTPS (Hypertext Transfer Protocol Secure) protocol, FHIR resources can be retrieved and parsed by analytics platforms for real-time data gathering. In this concept, healthcare organizations would be able to gather real-time data from specified resource models. FHIR resources can be streamed to a data repository where they can be correlated with other informatics data, such as medical imaging data outlined above. FHIR could facilitate epidemic tracking, prescription drug fraud, adverse drug interaction warnings, and the reduction of emergency room wait times.

As outlined above, when needed, appropriate medical imaging data can be obtained based on semantic information associated with the medical imaging data. Accordingly, as an example, when a catheter lab nurse or a cardiologist would like to study/check a specific instance of medical imaging data of a patient which depicts the narrowest position of the left coronary artery of the patient, the catheter lab nurse or the cardiologist could obtain the appropriate slice of CTA image or the appropriate visualization of the left coronary artery based on semantic information, e.g., the following keywords "narrowest", "left coronary artery", and "John Smith", i.e., the name of the patient. As such, there is no need to involve time-consuming manual browsing of DICOM studies & series.

According to various examples, various options are disclosed for obtaining the semantic information.

According to some examples, it would be possible that the semantic information is obtained based on a subscription. The subscription can generally define certain circumstances under which a push notification is triggered by an update of the semantic information at a respective data repository. Here, a subscriber may be proactively notified upon semantic information becoming available. The semantic information that becomes newly available can be received at the user node in a push notification. Such a scenario enables to maintain an up-to-date record of relevant semantic information at a user node. Thereby, lead time to obtain the semantic information can be reduced.

According to further examples, it would be possible that obtaining the semantic information includes retrieving, in a pull message, the semantic information based on the patient information. Thereby, it would be possible to actively query for the semantic information. A check for updates can be triggered by a user node. For instance, the pull message could be triggered whenever an update of the patient information at a local data repository and/or by a milestone of a patient management workflow occurs. This means that at certain situations of patient handling a query for the pull message can be triggered. For instance, the pull message could be queried and received in a background task. This means that no active user interaction may be required. Thereby, it is possible to obtain the semantic information in an event-based manner in accordance with patient handling, e.g., at a hospital.

By such events-triggered and/or subscription-triggered provisioning of the semantic information, it is possible to ensure that medical imaging data can be readily retrieved and, if necessary, post-processed. Specifically, scenarios are conceivable where post-processing of the medical imaging data that has been retrieved based on the semantic information requires significant time and/or is computationally expensive. It would be possible to implement such obtaining of the semantic information, obtaining of at least one instance of the medical imaging data and, optionally, post-processing of the medical imaging data in an end-to-end background task. Thereby, information that is extracted from the medical imaging data based on the postprocessing can be available with no latency to a medical practitioner, because the background task has been pre-executed and the respective information is readily available. This facilitates smooth patient handling in a patient management workflow at a hospital. Lead times to analyze and/or retrieve medical imaging data or other patient - related information such as the semantic information can be avoided.

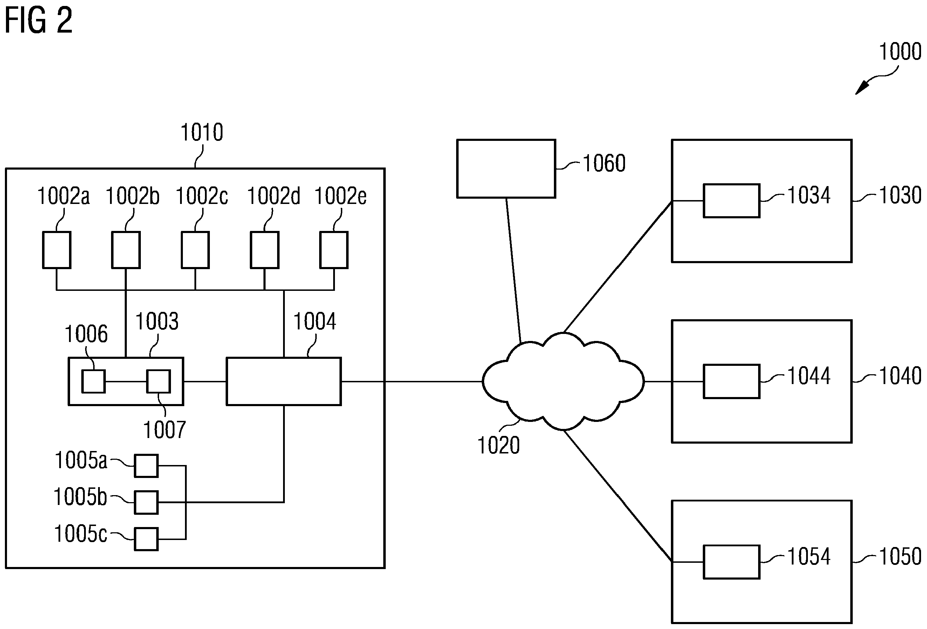

FIG. 2 schematically illustrates details with respect to a system 1000 according to various examples. The system 1000 may include four local networks 1010, 1030, 1040, as well as 1050, which are respectively within four hospitals or institutions, and a(n) external/shared network 1020, such as the internet or a cloud, via which the four local networks 1010, 1030, 1040, as well as 1050 may be communicated with each other. FIG. 1 is only an illustration of one possible example; generally, the number of local networks may be any positive integer, e.g., 1, 2, 3 and so on. The external/shared network 1020 is optional.

Each of the four local networks 1010, 1030, 1040, as well as 1050 may share the same or similar architecture and have the same or similar network elements or devices. For example, the local network 1010 may include at least one medical imaging equipment 1002a-1002e, at least one local data center 1003 comprising a PACS 1006 and a semantic database 1007 for storing semantic information associated with medical imaging data generated by the medical imaging equipment 1002a-1002e, at least one computing device 1004 connectable to the external/shared network 1020. The computing device 1004 can act as a gateway node to connect to the outside of the local network 1010, e.g., to any network node connectable via the external/shared network 1020. For example, the computing device 1004 can connect, via the external/shared network 1020, to respective computing devices 1034, 1044, and 1054 of respective local networks 1030, 1040, and 1050. Similarly, the respective computing devices 1034, 1044, and 1054 can also act as respective gateway nodes of the respective local networks 1030, 1040, and 1050. The local network 1010 further includes at least one user terminal 1005a-1005c, which is generally optional. Within the local network 1010, each of the at least one medical equipment 1002a-1002e is respectively connectable to the at least one local data center 1003, e.g., the PACS 1006 and the semantic database 1007, and the at least one computing device 1004 via physical cables or via wireless communication; each of the at least one user terminal 1005a-1005c may be connectable to the at least one computing device 1004 via physical cables or via wireless communication.

According to various examples, the medical imaging equipment 1002a-1002e includes one or more of an X-ray scanner, a computed tomography scanner, a magnetic resonance imaging scanner, a positron emission tomography scanner, an ultrasound scanner, and so on. A medical imaging examination of a patient can be performed by a radiologist using at least one of the medical imaging equipment 1002a-1002e, with respect to at least one anatomical region of the patient. Medical imaging data are obtained by the medical imaging examination and may be encoded according to a standard, such as the Digital Imaging and Communications in Medicine (DICOM) standard. Other standards, such as JPEG or TIFF, may be used. The (encoded) medical imaging data may be transmitted to the at least one local data center 1003 and/or the at least one computing device 1004. The (encoded) medical imaging data may be stored in the at least one local data center 1003 and/or in the at least one computing device 1004.

According to this disclosure, semantic information may be produced. As a general rule, various options for generating/producing these information are conceivable. For example, the semantic information that is associated with medical imaging data may be produced by a radiologist (and/or other medical practitioners) during a medical imaging examination of a patient, such as an ultrasound examination or an angiography examination. Additionally or alternatively, the semantic information may be produced by the radiologist (and/or other medical practitioners) after the medical imaging examination by reviewing/studying the medical imaging data acquired during the medical imaging examination. For example, the radiologist may obtain, via one of the at least one user terminal 1005a-1005c, the medical imaging data from the at least one local data center 1003, e.g., the PACS 1006, or the at least one computing device 1004, and compile the semantic information. In yet a further example, alternatively or additionally, the semantic information associated with medical imaging data could be extracted using computer image analysis algorithms running on each of the at least one medical imaging equipment 1002a-1002e, or on the at least one computing device 1004. I.e., a computerized implemented automated generation of the semantic information would be possible. For example, such computer image analysis algorithms could be integrated with or generally tied to medical imaging data acquisition algorithms, and thereby upon an instance of medical imaging data being acquired, a piece of semantic information associated with the acquired instance of medical imaging data is extracted using at least one appropriate computer image analysis algorithm. After producing/compiling the semantic information, the semantic information may be stored in the semantic database 1007. To facilitate efficiently and precisely obtaining a specific instance of medical imaging data, each instance of medical imaging data may have a unique identifier indicating a storage location of the respective instance of medical imaging data and such a unique identifier may be included in the respective semantic information. Accordingly, such a unique identifier could facilitate the efficiency and accuracy of obtaining an appropriate instance of medical imaging data of a patient. Other techniques can also be used to link respective semantic information stored in the semantic database 1007 to respective medical imaging data stored in the PACS 1006, such as a lookup table, or a codebook.

According to various examples, when a medical staff from the surgery department, the cardiology department, or the neurology department of a hospital where the local network 1010 is located, needs to obtain at least one instance of medical imaging data of a patient for a specific clinical practice, the medical staff could send a query message to the semantic database 1007 via any one of the user terminals 1005a-1005c to obtain semantic information associated with at least one instance of medical imaging data of a patient based on the patient information, which may include a link to the at least one instance of medical imaging data. Similarly, a medical staff from one of the other hospitals where the local networks 1030, 1040, and 1050 are located, needs to obtain the at least one instance of medical imaging data acquired in the hospital where the local network 1010 is located, the medical staff could send a query message to the computing device 1004, via a user terminal in the respective local networks 1030, 1040, and 1050 and connected to the respective computing devices 1034, 1044, and 1054. Upon receiving the query message, the computing device 1004 could configure a new query message and send it to the semantic database 1007 to obtain the at least one instance of medical imaging data.

Optionally, the system 1000 may include a central computing device 1060 (e.g., a remote server) which may be accessible to at least the respective computing devices 1004, 1034, 1044, and 1054 of the respective local networks 1010, 1030, 1040, and 1050. In some examples, each pair of the computing devices 1004, 1034, 1044, and 1054 may not be connectable directly, but can exchange data/information via the central computing device 1060, and thereby security of data generated/stored in the respective local networks 1010, 1030, 1040, and 1050 can be improved, for example by implementing access control techniques at the central computing device 1060. Central computing device 1060 also implement background tasks.

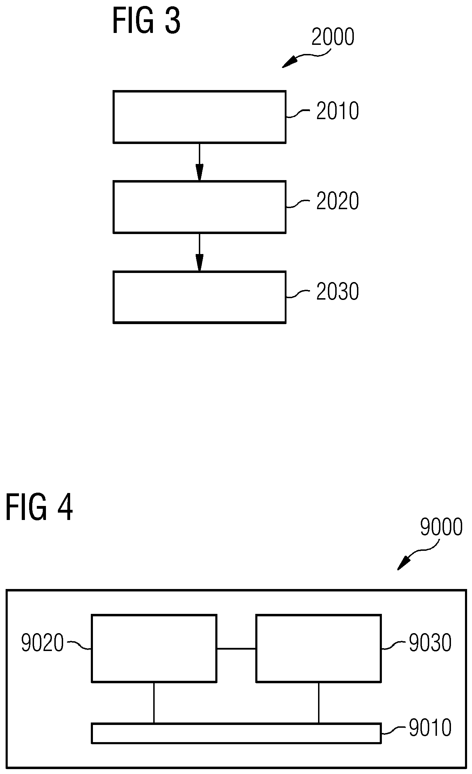

According to the disclosure, a method of operating a user node is provided. For example, as shown in FIG. 1, the user node could be any one of the user terminals 1005a-1005c in the local network 1010 or a similar user terminal (not shown in FIG. 1) in any one of the local networks 1030, 1040, and 1050. The method operates the user node to obtain at least one instance of medical imaging data of a patient based on semantic information associated with the at least one instance of the medical imaging data of the patient. The semantic information is obtained from a first data repository of a network, e.g., the semantic database 1007 of FIG. 1, based on patient information of the patient. Details with respect to such a method will be explained in connection with FIG. 3.

FIG. 3 is a flowchart of a method 2000 according to various examples. The method 2000 pertains to operating a user node to obtain at least one instance of medical imaging data of a patient.

The method 2000 can be executed by at least one processor upon loading program code. For example, the method 2000 could be executed by a processor of any one of the user terminal 1005a-1005c, upon loading program code from a respective memory.

At box 2010, patient information of a patient is obtained. For example, the patient information can be informed by the patient and input via one of the user terminals 1005a-1005c. Alternatively, the patient information may be obtained, via a smart card reader connected to one of the user terminals 1005a-1005c, by communicating with a smart card storing the patient information, such as an insurance card of the patient.

At box 2020, semantic information associated with at least one instance of medical imaging data of the patient is obtained from a first data repository of a network based on the patient information. Details with respect to obtaining the semantic information associated with at least one instance of medical imaging data of the patient will be explained in connection with the following two optional examples, i.e., subscription and retrieval.

### Example 1: Subscription

Said obtaining of the semantic information may include establishing a subscription to the semantic information associated with the at least one instance of the medical imaging data of the patient based on the patient information; and receiving, from the first data repository and in accordance with the subscription, the semantic information in a push notification triggered by an update of the semantic information at the first data repository. For example, a general practitioner (or family doctor) of a patient may subscribe semantic information of the patient in the respective semantic database of one or more medical centers. Accordingly, the general practitioner could timely obtain (new) health information of the patient and/or (new) medical imaging data acquired in the respective medical centers and thereby facilitate the following treatments. As another example, if an arbitrary ML algorithm detected an abnormality or a suspicious finding basically any software running on any user node could subscribe to such information and receive a notification to trigger alerts/action on their end. In such a scenario, it is not required to actively query for the semantic information. Rather, the semantic information may be proactively provided upon becoming available.

In general, the subscription to the semantic information associated with the at least one instance of the medical imaging data of the patient could particularly facilitate interoperability of healthcare information communication between different hospitals/institutions/clinics.

According to various examples, the subscription may include one or more filter criteria for filtering which type of the semantic information being updated triggers the push notification. This means that one or more filter criteria can define the circumstances and/or the type of semantic information for which a push notification is triggered.

For instance, one or more filter criteria can specify that certain semantic information pertaining to the specific body parts or specific diagnoses will be proactively provided in a push notification. The one or more filter criteria could specify that certain semantic information pertaining to specific body parts or diagnoses is excluded from push notifications.

The one or more filter criteria can explicitly or implicitly specify such positive or negative trigger criteria for proactively providing the push notification triggered by the update of the semantic information.

The one or more filter criteria may be defined based on a set of predefined medical proximity relationships between different types of the semantic information. For example, in terms of an individual patient, the one or more filter criteria may be defined based on a name of a disease and thereby semantic information extracted from different imaging modalities may be subscribed together. For example, CTA examination, MRI examination, and X-ray angiography examination may respectively be carried out for a patient suffering from coronary diseases. On the other hand, in terms of a group of patients, the one or more filter criteria may be defined based on a range of age, gender, or race.

By using medical proximity relationships, it is possible to obtain semantic information in a broad range of situations, pertaining, e.g., to a specific disease or pathology, without having to specify explicitly all respective imaging modalities, etc.

According to various examples, the one or more filter criteria may be determined based on one or more medical tasks associated with the patient and a predefined mapping from medical tasks to filter criteria.

### Example 2: Retrieval

Said obtaining of the semantic information may include retrieving, in a pull message and from the first data repository, the semantic information based on the patient information.

Thus, it would be possible to proactively query the semantic information.

According to various examples, the pull message may be triggered by an update of the patient information at a local data repository and/or meeting/reaching a predefined milestone of a patient management workflow. For instance, if a patient is registered for examination at a certain imaging device, it would be possible to automatically check - using the pull message - whether new up-to-date semantic information is available for the patient. For instance, if a patient checks in at the hospital, it would be possible to check for the up-to-date semantic information. For instance, certain milestones of a clinical workflow/patient management workflow, it would be possible to check whether the up-to-date semantic information is available.

For instance, depending on the type of the update of the patient information and/or depending on a type of the predefined milestone that triggers to query for the pull message, it would be possible to query for different semantic information. The milestone can be associated with a specific medical task, e.g., preparation for examination at a certain imaging modality, post-operative care, patient transfer between departments, to give just a few examples. For instance, if a patient is prepared for a CT chest examination, different semantic information may be queried if compared to a patient that is prepared for an MRI spine examination. Such mappings between type of update of the patient information and/or type of milestone of the patient management workflow, to semantic information that is retrieved and the pull message upon a respective query can be predefined by the user. Again, it would be possible to rely on medical proximity relationships in this regard.

Alternatively, the pull message may be queried using a subscription identity of the subscription according to Example 1. This would mean that an identifier and/link to the existing subscription may be provided, to check whether as part of the subscription, new updates are available.

According to various examples, the semantic information may be obtained from the first data repository based on a search query, the search query comprising at least one of an identity of the patient, an imaging modality of the medical imaging data, or a medical task associated with the patient.

By including such information in the search query, it is possible to obtain tailored semantic information. I.e., semantic information that is of relevance in accordance with a respective patient management workflow, more specifically of relevance with a current progress of the patient management workflow or a specific milestone of the patient management workflow, may be obtained. Here, it would be possible to consider one or medical proximity relationships between different types of semantic information and medical tasks or imaging modalities indicated by the search query. For instance, depending on the particular examination or diagnosis task, different types of semantic information may be retrieved in accordance with the medical proximity relationships. For instance, where a chest CT examination is to be performed, relevant semantic information that can describe pathologies that have impact on or can be diagnosed by the respective CT scans can be obtained.

For example, a query message could be sent to the semantic database 1007 via one of the user terminals 1005a-1005c. The query message may be configured based on the name of the patient, e.g., John Smith, and an imaging modality, e.g., MRI, CT, or CTA. Upon receiving the query message, the semantic database 1007 may provide/send sematic information which may, for example, be related to MRI imaging data, to the user terminal 1005a-1005c.

Optionally, the medical imaging data may include multiple instances of the medical imaging data and the semantic information associated with the medical imaging data may include individual semantic information associated with each of the multiple instances. Said obtaining of the semantic information may include obtaining respective individual semantic information associated with one or more instances of the medical imaging data; and said obtaining of the medical imaging data may include obtaining the one or more instances of the medical imaging data based on the respective individual semantic information.

At box 2030, the at least one instance of the medical imaging data is obtained based on the semantic information associated with the at least one instance of medical imaging data of the patient.

Box 2030 can be triggered by new semantic information being obtained at box 2020. Thus, it would be possible that medical imaging data is obtained automatically upon receiving a push notification at box 2020, the push notification including the semantic information. Thus, it is possible that the medical imaging data is obtained in a background task without requiring user interaction.

For examples, the semantic information may include an identifier indicating a storage location of the at least one instance of the medical imaging data on the network and the at least one instance of the medical imaging data may be accordingly obtained based on the identifier.

Optionally, the semantic information may be structured based on a pre-configured data model and the identifier may be obtained by parsing the semantic information, based on the pre-configured data model. For example, the semantic information including the identifier could be structured using XML, i.e., storing as an XML format file, and thereby the identifier can be obtained by parsing the XML format file.

For examples, the at least one instance of the medical imaging data may be stored in a second data repository, e.g., the semantic database 1007 of FIG. 1, of the network and the identifier may include a uniform resource locator associated with the second data repository. The uniform resource locator may be a reference to the medical imaging data that may specify its location on a network and a mechanism for retrieving it.

Once desired medical imaging data are obtained, the desired medical imaging data and/or the semantic information associated with the desired medical imaging data could facilitate various clinical practices or workflows. The method 2000 may optionally further include processing the semantic information and the at least one instance of the medical imaging data according to different clinical practices/workflows, such as adjusting parameters of a medical imaging equipment based on the semantic information and the at least one instance of the medical imaging data; determining a diagnosis based on the semantic information and the at least one instance of the medical imaging data; or developing a treatment plan based on the semantic information and the at least one instance of the medical imaging data.

The method 2000 facilitate automatically obtaining desired medical imaging data of a patient based on semantic information associated with the desired medical imaging data and thereby makes clinical information, i.e., semantic information extracted from medical imaging data and corresponding relevant imaging data, available to clinical experts and other Healthcare IT software products and machines along the whole clinical pathways to improve quality of care, workflow efficiency, and cost. Accordingly, interoperability among elements of healthcare system is improved. In particular, it is possible to facilitate interoperability between legacy health care systems, to make it easy to provide health care information to health care providers and individuals on a wide variety of devices from computers to tablets to cell phones and to allow third-party application developers to provide medical applications which can be easily integrated into existing systems. Advantages of the method 2000 could be reflected in the following use cases.

### Radiology internal example:

Patient shall receive an oncologic follow-up scan. The goal is that the CT scan software matches the image impression of the prior scan nicely to make the images more comparable. Therefore, an easy way of searching and identifying all prior results is obtaining semantic information associated with CT scans from the semantic database to obtain all prior CT imaging data. Then CT scan software can load the DICOM headers of prior CT scans and retrieve scan parameters from it to match acquisition parameters to the prior exam.

### Cardiology example:

The angiography system shall auto-load and display CT images. Therefore, for each patient treated in the catheter lab, the C-arm X-ray machine shall query the semantic database if CT images exist in the PACS. The semantic database may return a list of DICOM instance references which then can be loaded and displayed by the C-arm X-ray machine fully automatically.

FIG. 4 is a block diagram of a device 9000 according to various examples. The device 9000 may include at least one processor 9020, at least one memory 9030, and at least one input/output interface 9010. The at least one processor 9020 is configured to load program code from the at least one memory 9030 and execute the program code. Upon executing the program code, the at least one processor 9020 performs the method 2000.

Summarizing, techniques have been described that facilitate automatically obtaining desired medical imaging data of a patient based on semantic information associated with the desired medical imaging data and thereby makes clinical information, i.e., semantic information extracted from medical imaging data and corresponding relevant imaging data, available to clinical experts and other healthcare IT software products and machines, e.g., medical imaging devices, along the whole clinical pathways to improve quality of care, workflow efficiency, and cost. Accordingly, interoperability among elements of healthcare system is improved. In particular, it is possible to facilitate interoperability between legacy health care systems, to make it easy to provide health care information to health care providers and individuals on a wide variety of devices from computers to tablets to cell phones and to allow third-party application developers to provide medical applications which can be easily integrated into existing systems.

Although the disclosure has been shown and described with respect to certain preferred embodiments, equivalents and modifications will occur to others skilled in the art upon the reading and understanding of the specification. The present disclosure includes all such equivalents and modifications and is limited only by the scope of the appended claims.

## Claims

1. A computer-implemented method of operating a user node (1002a-1002e), the method comprising:
- obtaining (2010) patient information of a patient;
- obtaining (2020), from a first data repository (1007) of a network (1010), semantic information associated with at least one instance of medical imaging data of the patient based on the patient information;
- obtaining (2030) the at least one instance of the medical imaging data based on the semantic information.

2. The computer-implemented method of claim 1,
wherein said obtaining of the semantic information comprises:
- establishing a subscription to the semantic information associated with the at least one instance of the medical imaging data of the patient based on the patient information; and
- receiving, from the first data repository (1007) and in accordance with the subscription, the semantic information in a push notification triggered by an update of the semantic information at the first data repository (1007).

3. The computer-implemented method of claim 2,
wherein the subscription comprises one or more filter criteria for filtering which type of the semantic information being updated triggers the push notification.

4. The computer-implemented method of claim 3,
wherein the one or more filter criteria are defined based on a set of predefined medical proximity relationships between different types of the semantic information.

5. The computer-implemented method of claim 3 or 4,
wherein the one or more filter criteria are determined based on one or more medical tasks associated with the patient and a predefined mapping from medical tasks to filter criteria.

6. The computer-implemented method of claim 1,
wherein said obtaining of the semantic information comprises:
- retrieving, in a pull message and from the first data repository (1007), the semantic information based on the patient information.

7. The computer-implemented method of claim 6,
wherein the pull message is triggered by at least one of an update of the patient information at a local data repository or a predefined milestone of a patient management workflow.

8. The computer-implemented method of claim 6 or 7, and of any one of claims 2 to 5,
wherein the pull message is queried using a subscription identity of the subscription.

9. The computer-implemented method of any one of the preceding claims,
wherein the semantic information is obtained from the first data repository (1007) based on a search query, the search query comprising at least one of an identity of the patient, an imaging modality of the medical imaging data, or a medical task associated with the patient.

10. The computer-implemented method of any one of the preceding claims,
wherein the semantic information comprises an identifier indicating a storage location of the at least one instance of the medical imaging data on the network (1010).

11. The computer-implemented method of claim 10,
wherein the semantic information is structured based on a pre-configured data model,
the method further comprising:
- parsing the semantic information, based on the pre-configured data model, to obtain the identifier.

12. The computer-implemented method of claim 10 or 11,
wherein the at least one instance of the medical imaging data is stored in a second data repository (1006) of the network (1010) and the identifier comprises a uniform resource locator associated with the second data repository (1006) .

13. The computer-implemented method of any one of the preceding claims, further comprising:
processing at least one of the semantic information or the at least one instance of the medical imaging data,
wherein said processing of the semantic information and the at least one instance of the medical imaging data comprises at least one of:
adjusting parameters of a medical imaging equipment based on the semantic information and the at least one instance of the medical imaging data;
determining a diagnosis based on the semantic information and the at least one instance of the medical imaging data; and
developing a treatment plan based on the semantic information and the at least one instance of the medical imaging data.

14. The computer-implemented method of claim 13,
wherein said obtaining of the semantic information, said obtaining of the at least one instance of the medical imaging data, and said processing of the at least one of the semantic information or the at least one instance of the medical imaging data is performed in an end-to-end background process without user interaction.

15. A device (9000), the device (9000) comprising at least one processor (9020) and the at least one processor (9020) being configured to:
- obtain (2010) patient information of a patient;
- obtain (2020), from a first data repository (1007) of a network (1010), semantic information associated with at least one instance of medical imaging data of the patient based on the patient information;
- obtain (2030) the at least one instance of the medical imaging data based on the semantic information.

## Amended claims

### Amended claims in accordance with Rule 137(2) EPC.

1. A computer-implemented method of operating a user node (1002a-1002e), the method comprising:
- obtaining (2010) patient information of a patient;
- obtaining (2020), from a first data repository (1007) of a network (1010), semantic information associated with at least one instance of medical imaging data of the patient based on the patient information, wherein the semantic information comprises an identifier indicating a storage location of the at least one instance of the medical imaging data on the network (1010);
- obtaining (2030) the at least one instance of the medical imaging data based on the semantic information.

2. The computer-implemented method of claim 1,
wherein said obtaining of the semantic information comprises:
- establishing a subscription to the semantic information associated with the at least one instance of the medical imaging data of the patient based on the patient information; and
- receiving, from the first data repository (1007) and in accordance with the subscription, the semantic information in a push notification triggered by an update of the semantic information at the first data repository (1007).

3. The computer-implemented method of claim 2,
wherein the subscription comprises one or more filter criteria for filtering which type of the semantic information being updated triggers the push notification.

4. The computer-implemented method of claim 3,
wherein the one or more filter criteria are defined based on a set of predefined medical proximity relationships between different types of the semantic information.

5. The computer-implemented method of claim 3 or 4,
wherein the one or more filter criteria are determined based on one or more medical tasks associated with the patient and a predefined mapping from medical tasks to filter criteria.

6. The computer-implemented method of claim 1,
wherein said obtaining of the semantic information comprises:
- retrieving, in a pull message and from the first data repository (1007), the semantic information based on the patient information.

7. The computer-implemented method of claim 6,
wherein the pull message is triggered by at least one of an update of the patient information at a local data repository or a predefined milestone of a patient management workflow.

8. The computer-implemented method of claim 6 or 7, and of any one of claims 2 to 5,
wherein the pull message is queried using a subscription identity of the subscription.

9. The computer-implemented method of any one of the preceding claims,
wherein the semantic information is obtained from the first data repository (1007) based on a search query, the search query comprising at least one of an identity of the patient, an imaging modality of the medical imaging data, or a medical task associated with the patient.

10. The computer-implemented method of any one of the preceding claims,
wherein the semantic information is structured based on a pre-configured data model,
the method further comprising:
- parsing the semantic information, based on the pre-configured data model, to obtain the identifier.

11. The computer-implemented method of any one of the preceding claims,
wherein the at least one instance of the medical imaging data is stored in a second data repository (1006) of the network (1010) and the identifier comprises a uniform resource locator associated with the second data repository (1006).

12. The computer-implemented method of any one of the preceding claims, further comprising:
processing at least one of the semantic information or the at least one instance of the medical imaging data,
wherein said processing of the semantic information and the at least one instance of the medical imaging data comprises at least one of:
adjusting parameters of a medical imaging equipment based on the semantic information and the at least one instance of the medical imaging data;
determining a diagnosis based on the semantic information and the at least one instance of the medical imaging data; and
developing a treatment plan based on the semantic information and the at least one instance of the medical imaging data.

13. The computer-implemented method of claim 12,
wherein said obtaining of the semantic information, said obtaining of the at least one instance of the medical imaging data, and said processing of the at least one of the semantic information or the at least one instance of the medical imaging data is performed in an end-to-end background process without user interaction.

14. A device (9000), the device (9000) comprising at least one processor (9020) and the at least one processor (9020) being configured to:
- obtain (2010) patient information of a patient;
- obtain (2020), from a first data repository (1007) of a network (1010), semantic information associated with at least one instance of medical imaging data of the patient based on the patient information, wherein the semantic information comprises an identifier indicating a storage location of the at least one instance of the medical imaging data on the network (1010);
- obtain (2030) the at least one instance of the medical imaging data based on the semantic information.

15. A computer program including program code, wherein the program code can be loaded and executed by at least one processor, wherein upon loading and executing the program code, the at least one processor performs a computer-implemented method according to one of the claims 1 to 13.
